# EUROPEAN PATENT APPLICATION

(11) **EP 4 831 638 A1**
(43) Date of publication of application: **28.10.2026**
(21) Application number: 25382404.9
(22) Date of filing: 22.04.2025
(51) Int. Cl.: G01N 33/68

(54) **IN VITRO METHOD FOR ASSESSING OXIDATIVE STRESS**

(71) Applicant: Servizo Galego De Saúde, 15703 Santiago de Compostela, A Coruna (ES); Universidade de Santiago de Compostela (USC), 15872 Santiago de Compostela A Coruña (ES); Fundación Pública Galega Instituto de Investigación Sanitaria de Santiago de Compostela, 15706 Santiago de Compostela A Coruña (ES)
(72) Inventor: CRUCES SANDE, Antón, 15706 Santiago de Compostela (ES); VÁZQUEZ AGRA, Néstor, 15706 Santiago de Compostela (ES); HERMIDA AMEIJEIRAS, Álvaro, 15706 Santiago de Compostela (ES); MÉNDEZ ÁLVAREZ, Estefanía, 15872 Santiago de Compostela (ES); SOTO OTERO, Ramón, 15872 Santiago de Compostela (ES)
(74) Representative: Hoffmann Eitle

(57) **Abstract**

***IN VITRO* METHOD FOR ASSESSING OXIDATIVE STRESS.** The present invention refers to an *in vitro* method for assessing oxidative stress and/or for the diagnosis of hypertension and/or cardiovascular disease in a urine sample obtained from the subject.

## Description

### FIELD OF THE INVENTION

The present invention refers to the medical field. Particularly, the present invention refers to an *in vitro* method for assessing oxidative stress and/or for the diagnosis of hypertension and/or cardiovascular disease (CVD) in a urine sample obtained from the subject.

### STATE OF THE ART

Oxidative stress is a condition characterized by an imbalance between the production of oxidizing species-notably oxygen species (ROS)-and the body's ability to counteract their effects through antioxidant defence and repair mechanisms.

This imbalance can lead to cellular damage, playing a significant role in the development and progression of CVDs, particularly hypertension and atherosclerosis. In cardiovascular health, oxidative stress damages proteins, lipids, and DNA in vascular cells, accelerating the decline of their function. At a more systemic level, this translates into impaired nitric oxide bioavailability, endothelial dysfunction, inflammation, and arterial stiffness. These consolidated effects of oxidative stress on the cardiovascular system are key drivers in the development and progression of CVD, notably hypertension and atherosclerosis.

Two important biomarkers involved in oxidative stress are Thiobarbituric Acid Reactive Substances (TBARS) and reduced thiols. TBARS are a group of compounds formed as a result of lipid peroxidation, a process where ROS interact with cell membrane lipids, particularly polyunsaturated fatty acids. This reaction results in the formation of aldehydes, such as malondialdehyde (MDA), which can then react with thiobarbituric acid to form a colored complex. The presence of TBARS, particularly MDA, is often used to indicate lipid damage due to oxidative stress, as they reflect the extent of lipid peroxidation in cells and tissues.

Thiols (-SH), on the other hand, are sulfur-containing compounds that play a key role in maintaining the cellular redox state. The most common thiol in the human body is glutathione (GSH), which, upon oxidation, forms glutathione disulfide (GSSG). Thiols (often used interchangeably with reduced thiols) are critical for neutralizing ROS and maintaining cellular antioxidant defenses. The levels of reduced thiols in tissues and fluids reflect the capacity of the body to counteract oxidative stress. A decrease in thiol levels indicates an overwhelmed antioxidant system, suggesting that oxidative damage is occurring.

Both TBARS and thiols are intrinsically linked to oxidative processes within the body and are indicative of cellular health. When oxidative stress occurs, the extent of TBARS formation and thiol depletion can serve as indicators of cellular damage and the body's oxidative response capacity.

In the context of CVDs, monitoring oxidative stress is crucial not only for understanding the underlying mechanisms of disease progression but also for assessing disease severity, stratifying risk, and improving cardiovascular risk evaluation. However, traditional methods of assessing oxidative stress often require invasive procedures, such as blood sampling, which can be burdensome for patients, especially in cases where regular monitoring is needed.

To overcome these limitations, there is a growing interest in exploring alternative, non-invasive sampling methods.

The development of reliable, non-invasive methods for assessing oxidative stress could revolutionize the management of these diseases, enabling more frequent monitoring, early detection, and improved patient outcomes without the need for invasive procedures.

### DECRIPTION OF THE INVENTION

### Brief description of the invention

The present invention refers to an *in vitro* method for assessing oxidative stress and/or for the diagnosis of hypertension and/or cardiovascular disease in urine samples obtained from the subject.

Particularly, the present invention provides an *in vitro* method for assessing oxidative stress in a subject by identifying and/or quantifying TBARS and/or thiols in urine samples.

Oxidative stress is a relevant factor in cardiovascular risk assessment, as it contributes to disease mechanisms and progression. This method offers a non-invasive, practical, and reliable approach for monitoring oxidative stress, which plays a key role in disease progression, diagnosis, and early intervention.

TBARS are compounds formed as a result of lipid peroxidation, a process in which reactive oxygen species (ROS) attack cell membranes, particularly polyunsaturated fatty acids. The resulting products of lipid oxidation, including malondialdehyde (MDA), form TBARS. Elevated TBARS levels are indicative of oxidative stress, which is implicated in a range of diseases, especially cardiovascular conditions such as hypertension and atherosclerosis. In the method herein described, TBARS are identified and/or quantified by reacting the urine sample with thiobarbituric acid (TBA) under acidic conditions and 95°C to form a colored complex. The absorbance of this complex is directly proportional to the TBARS concentration, allowing for a reliable and quantitative measurement of lipid peroxidation and oxidative stress.

Thiols-normally synonymous with reduced or free thiols-are key antioxidants that protect cells from oxidative damage by neutralizing ROS. The most abundant reduced thiol in biological systems is glutathione (GSH), which plays a central role in maintaining redox homeostasis. The concentration of reduced thiols in a biological sample reflects the body's ability to counteract oxidative stress. A decrease in thiol levels suggests an overwhelmed antioxidant defense system, indicative of oxidative damage. In this invention, thiols are quantified by reacting the urine sample with 5,5'-dithiobis(2-nitrobenzoic acid) (DTNB) under physiological pH conditions to form a colored complex. The absorbance of this complex is directly proportional to the reduced thiol concentration, providing a reliable and quantitative measurement of antioxidant status and redox balance.

This invention also contributes to the assessment of cardiovascular diseases, including hypertension and atherosclerosis, where oxidative stress-reflected by altered TBARS and/or thiol levels-plays a significant role in disease pathogenesis. The method of the invention can be used to assess oxidative stress and its contribution to these conditions. By comparing the levels of TBARS and/or thiols to established thresholds from healthy subjects or those without oxidative stress, the method provides an efficient tool for improving cardiovascular risk assessment and stratification.

The use of urine samples provides several advantages over traditional invasive methods such as blood/plasma tests. Urine collection is non-invasive, easily accessible, and can be done repeatedly with minimal discomfort to the patient. This non-invasive nature of the method makes it especially advantageous in clinical settings, where frequent monitoring of oxidative stress can be crucial for disease prevention, early detection, and management.

Such as it is shown in **Example 2** below, a correlation greater than +0.7 was observed between plasma and urine samples, suggesting a strong positive correlation, meaning that, as the concentration of the substance in the plasma increases, it also tends to increase in the urine, and vice versa. This indicates that the levels of the substance in these two biological fluids are closely related and that changes in one could reflect similar changes in the other.

This strong correlation supports the use of urine samples as a substitute for plasma samples, since plasma samples are associated with several limitations:
- Sample collection: Plasma sampling requires a specific blood draw, an invasive procedure that is not without potential complications. It also necessitates the involvement of nursing staff for venipuncture and a centrifuge operator to process the sample immediately.
- Sample processing: Biochemical assays for plasma involve multiple steps to remove protein interferences, requiring a trained specialist to perform the procedure. Additionally, the workflow for plasma sample preparation typically takes between 3 to 4 hours, making it a more time-consuming process.

So, urine offers significant advantages over plasma because:
- It allows for a more convenient sample collection for the patient, eliminating the need for nursing intervention or a centrifuge operator.
- It is protein-free, enabling the development of an innovative method based on preloaded assay plates, which significantly shortens the workflow time, enhancing efficiency and throughput.

The use of urine samples enables the implementation of a preloaded plate system with stabilized reagents, revolutionizing oxidative stress assessment by simplifying sample preparation and enhancing efficiency. Unlike plasma, urine is naturally protein-free, eliminating the need for complex preprocessing steps such as protein precipitation and removal, which are essential in plasma assays to prevent unwanted interference. This key characteristic allows reagents to directly interact with the sample without additional purification steps, making urine an ideal biological fluid for preloaded assay systems.

Moreover, the absence of proteins in urine contributes to greater reagent stability. In plasma-based assays, proteins and other biomolecules can degrade reagents over time, necessitating their fresh preparation before each analysis. In contrast, urine's simpler biochemical composition ensures that reagents remain stable over extended periods, making it possible to develop preloaded and sealed plates that can be stored and used on demand. This feature significantly enhances the reproducibility and standardization of oxidative stress assays in clinical laboratories, minimizing variability between tests.

The ability to preload and seal reagents within a plate also allows for high-throughput processing, optimizing laboratory workflows. Traditional plasma-based methods often require extensive manual handling, significantly limiting throughput. In contrast, this new procedure increases sample processing capacity by an order of magnitude, enabling laboratories to analyze a substantially higher volume of samples in less time while maintaining precision and reliability.

Additionally, by replacing invasive blood draws with non-invasive urine sampling, this approach not only enhances patient comfort but also eliminates the need for specialized nursing staff and centrifuge operators, significantly reducing the workload of healthcare personnel. The transition to urine-based assays thus marks a pivotal advancement in oxidative stress evaluation, combining efficiency, stability, and ease of use to set a new standard in clinical diagnostics.

The urine results obtained in the present invention can be considered unexpected. Although this invention demonstrates a strong correlation between plasma and urine samples, the interchangeability between these two biological fluids is neither automatic nor obvious. Many biomarkers that are well-established in plasma fail to maintain the same stability, detectability, or diagnostic value in urine, often requiring significant methodological adaptations. The use of urine as an alternative for oxidative stress assessment is therefore not straightforward or trivial.

Despite the widespread use of plasma in diagnostics, urine had not previously been validated for systematic oxidative stress evaluation. If urine had been recognized as a reliable alternative, redox state assessment would have been conducted more frequently, without the limitations imposed by the invasive nature of plasma collection. The fact that such an approach has not been widely explored suggests that its feasibility was not intuitively evident.

From a physicochemical standpoint, several challenges make urine an unpredictable matrix for biomarker analysis. Unlike plasma, urine composition is highly variable and influenced by multiple physiological and biochemical factors, including glomerular filtration rate, tubular reabsorption, and secretion mechanisms. Different analytes are not filtered, reabsorbed, or excreted in the same proportions, meaning that a biomarker's presence in plasma does not guarantee a proportional or detectable presence in urine. Additionally, oxidative modifications can alter a molecule's physicochemical properties, such as polarity, charge, or solubility, potentially affecting its renal elimination. Some oxidized species may become more hydrophilic and be preferentially excreted, while others may bind to proteins, aggregate, or undergo further metabolic transformations that hinder their detection.

Moreover, to overcome the intrinsic variability of urine dilution, we applied a normalization step based on urinary creatinine concentration, ensuring a more accurate and reliable quantification of oxidative stress markers. This methodological decision was crucial to account for physiological differences in urine concentration across individuals and sampling conditions. In addition, for TBARS measurements in plasma, we adjusted the results by the concentration of plasma lipoproteins obtained from simultaneous routine blood analysis. This correction is essential because lipid peroxidation predominantly affects lipoprotein fractions, and without this normalization, comparisons across individuals or samples would be misleading. These normalization strategies, although analytically demanding, were necessary to obtain consistent and interpretable data, and further highlight the non-obviousness of translating oxidative stress assessment from plasma to urine.

Thus, adapting plasma-based oxidative stress biomarkers for urine analysis required a specific methodological approach to determine which markers could be reliably detected and quantified for cardiovascular risk assessment. Simply assuming that urine could substitute plasma without addressing these biochemical and physicochemical challenges would have been overly simplistic and technically unfounded.

On the other hand, to our knowledge, the prior art does not suggest or teach that urine could be substituted for plasma in the specific context of the present invention.

Finally, as explained above, the present invention provides clear improvements (e.g., higher accuracy, better stability or lower cost) enabling the implementation of a preloaded plate system with stabilized reagents, revolutionizing oxidative stress assessment by simplifying sample preparation and enhancing efficiency.

So, the first embodiment of the present invention refers to an *in vitro* method for assessing oxidative stress. In this embodiment, a urine sample is collected from a subject suspected of being under oxidative stress. The sample is then processed to identify and/or quantify TBARS and/or thiols. TBARS are preferably measured by reacting the urine sample with thiobarbituric acid (TBA) under acidic conditions and 95°C to form a colored complex, and the absorbance of the complex is measured. The absorbance value is directly proportional to the concentration of TBARS in the urine sample. Similarly, reduced thiols are preferably quantified by reacting the sample with 5,5'-dithiobis (2-nitrobenzoic acid), which forms a colored product. The degree of color change correlates to the concentration of reduced thiols in the urine. This method provides a reliable *in vitro* assessment of oxidative stress in subjects, allowing the clinician to gauge the level of oxidative damage in a non-invasive manner.

The second embodiment of the present invention refers to an in vitro method for the assessment of cardiovascular disease risk. The method of assessing oxidative stress as described above is applied to evaluate the risk of CVDs, such as hypertension or atherosclerosis. Following the identification and/or quantification of TBARS and/or thiols in the urine sample, the resulting oxidative stress levels are compared to established reference thresholds from healthy subjects. Elevated levels of TBARS or decreased levels of thiols are indicative of oxidative stress, which contribute to the development of CVD. This embodiment allows healthcare providers to utilize non-invasive urine samples for the diagnosis and monitoring of these conditions, providing a valuable tool for early detection and intervention.

The third embodiment of the present invention refers to the *in vitro* use of TBARS and/or thiols for assessing oxidative stress and/or diagnosing hypertension and/or cardiovascular disease. This embodiment describes the *in vitro* use of TBARS and/or thiols in a urine sample for assessing oxidative stress and diagnosing hypertension and cardiovascular disease. The method preferably involves reacting the urine sample with TBA and measuring the resulting complex's absorbance to quantify TBARS and using 5,5'-dithiobis (2-nitrobenzoic acid) to detect thiols. The use of this method in clinical settings provides an efficient, non-invasive means for monitoring patients with hypertension and cardiovascular diseases. This allows physicians to make informed decisions based on oxidative stress levels as a contributing factor to disease progression and therapeutic efficacy.

The fourth embodiment of the present invention refers to the identification and/or quantification of TBARS by reacting with TBA. In this embodiment, a urine sample from a subject is reacted with TBA under acidic conditions. The reaction produces a colored complex, the intensity of which is measured using a spectrophotometer. The absorbance of the complex correlates directly with the concentration of TBARS in the urine sample. The higher the absorbance, the higher the level of TBARS present, indicating a greater degree of oxidative stress in the subject. This embodiment provides a reliable and reproducible method for detecting oxidative damage in the form of TBARS.

The fifth embodiment of the present invention refers to the identification and/or quantification of thiols by reaction with 5,5'-dithiobis (2-nitrobenzoic acid). The embodiment involves reacting reduced thiols in a urine sample with 5,5'-dithiobis (2-nitrobenzoic acid). The reaction forms a colored product, and the intensity of the color change is measured spectrophotometrically. The color intensity is proportional to the concentration of reduced thiols in the sample. A decrease in reduced thiols suggests oxidative stress, which may indicate the presence of a disease condition like hypertension or CVD. This embodiment provides an efficient and straightforward method for quantifying thiols as part of the oxidative stress assessment.

The sixth embodiment of the present invention refers to the increased TBARS levels as an indication of oxidative stress. This embodiment involves measuring TBARS in the urine sample and comparing the results to a pre-established threshold value. The threshold value is derived from normal (non-oxidative stress) subjects. An increase in the levels of TBARS above this threshold indicates the presence of oxidative stress in the subject. This method can be used as a diagnostic tool for conditions such as hypertension, where oxidative stress is often implicated. A simple, non-invasive urine test can provide a quick and reliable indication of oxidative stress levels, aiding in the early detection and management of these diseases.

The seventh embodiment of the present invention refers to a decreased reduced thiols as an indication of oxidative stress. In this embodiment, reduced thiols are quantified in a urine sample and compared to a pre-established threshold value derived from healthy subjects. A decrease in the concentration of reduced thiols below this threshold serves as an indication of oxidative stress. The method of measuring thiols involves the reaction with 5,5'-dithiobis(2-nitrobenzoic acid), forming a colored product whose intensity is directly proportional to the thiol concentration. Reduced thiol levels lower than the threshold are associated with a compromised antioxidant defense system, which is a hallmark of oxidative stress and may indicate the presence of underlying conditions like hypertension or CVD.

The eight embodiment of the present invention refers to a preloaded plate system for oxidative stress assessment, comprising a plurality of wells preloaded with stabilized reagents selected from thiobarbituric acid and a thiol-detecting chromogenic compound, wherein said reagents are pre-sealed to maintain stability over an extended period, and wherein the system is configured to directly analyze urine samples without prior protein removal.

In a preferred embodiment, the thiol-detecting chromogenic compound is 5,5'-dithiobis-(2-nitrobenzoic acid) (DTNB).

In a preferred embodiment, the stabilized reagents are sealed within the wells of the plate under vacuum to preserve their stability during storage.

In a preferred embodiment, the system is designed to process up to 240 urine samples in a single workday.

In a preferred embodiment, the system eliminates the need for blood draws and reduces the workload of healthcare personnel.

In a preferred embodiment, the plate is compatible with automated or high-throughput analyzers for large-scale clinical use.

In a preferred embodiment, the system is designed for oxidative stress marker detection in urine without additional sample preparation beyond collection.

In a preferred embodiment, the thiol-detecting chromogenic compound is selected from a group consisting of 5,5'-dithiobis-(2-nitrobenzoic acid) (DTNB), Ellman's reagent, and other sulfhydryl-reactive chromogenic compounds.

For the purpose of the present invention the following terms are defined:
- The term "comprising" it is meant including, but not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of". Thus, the phrase "consisting of" indicates that the listed elements are required or mandatory, and that no other elements may be present.
- "Oxidative stress" refers to a physiological condition characterized by an imbalance between the production of reactive oxidizing species and the body's ability to neutralize them with its antioxidant defenses, leading to potential cellular damage.
- "TBARS" refers to a group of compounds formed during the peroxidation of lipids, typically measured as a marker of oxidative stress, and includes substances such as malondialdehyde (MDA).
- "Thiols" refers to compounds containing a sulfur-hydrogen (-SH) group, which are often involved in redox reactions and act as antioxidants in biological systems. The term "reduced thiols" is often used synonymously with "thiols" to emphasize their non-oxidized state, which is directly linked to antioxidant activity
- "Threshold value" refers to a pre-established value for TBARS or reduced thiols, typically derived from subjects without oxidative stress, which serves as a reference to indicate abnormal levels associated with oxidative stress. The threshold value for oxidative stress markers, such as TBARS or reduced thiols, can be calculated using methods known in the prior art or based on common general knowledge in the field. Specifically, threshold values may be derived from baseline measurements taken from healthy individuals or control groups, as described in existing literature. These values are typically used as reference points to determine the presence of oxidative stress in a subject by comparing the concentration of oxidative stress markers in the subject's sample to the established baseline threshold.
- "Reagents" refers to chemicals used to facilitate the detection and measurement of oxidative stress markers, such as thiobarbituric acid and thiol-detecting chromogenic compounds.

### Brief description of the figures

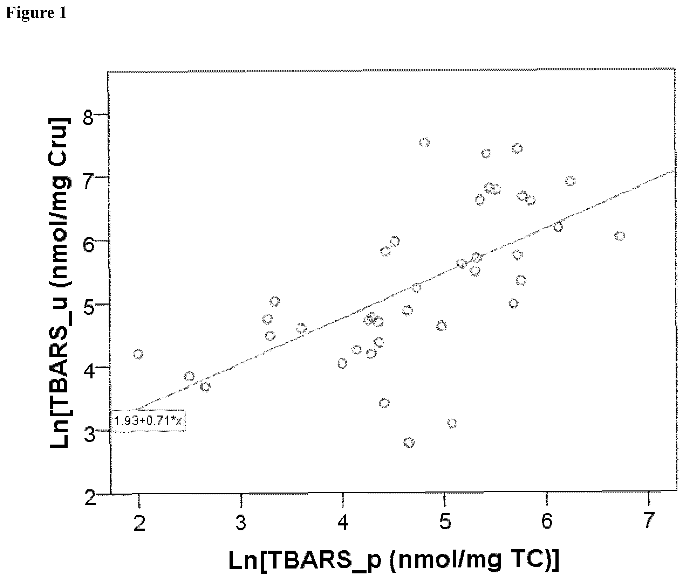

Figure 1. Linear correlation analysis between plasma-urine TBARS. Correlation equation: Ln[TBARSu] = 1.93 + 0.71 × Ln[TBARSp]. Spearman's rho = 0.717, p < 0.001. TBARS-Thiobarbituric acid reactive substances; TBARSu-Urinary TBARS; TBARSp-Plasmatic TBARS; Cru-Urinary creatinine; TC-Total cholesterol; Ln-Natural logarithm; nmol-Nanomole; mg-Milligram.

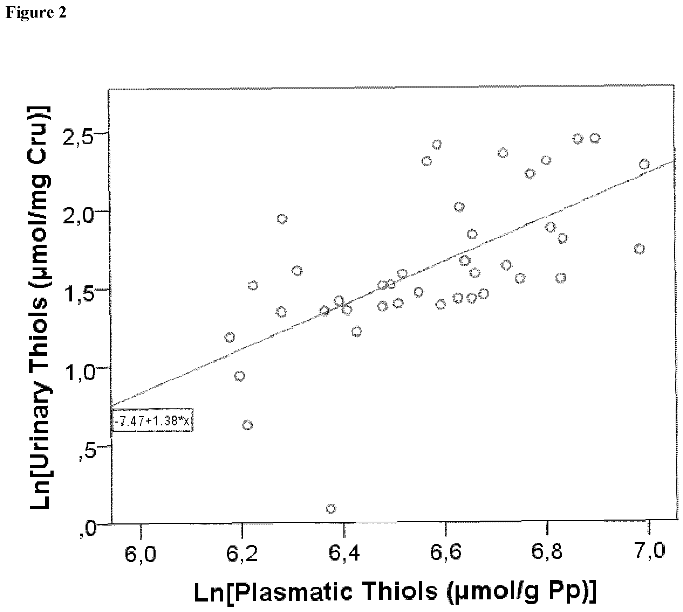

Figure 2. Linear correlation analysis between plasma-urine thiols. Correlation equation: Ln[Thiolsᵤ] = 1.93 + 0.71 × Ln[Thiolsₚ]. Spearman's rho = 0.697, p < 0.001. Thiolsᵤ-Urinary thiols; Thiolsₚ-Plasmatic thiols; Crᵤ-Urinary creatinine; Pₚ-Plasmatic proteins; Ln-Natural logarithm; umol-Micromole; mg-Milligram; g-Gram.

### Detailed description of the invention

The present invention is illustrated by means of the Examples set below, without the intention of limiting its scope of protection.

### Example 1. Material and methods

### Example 1.1. Design, Setup and Participants

This study was designed as an observational and paired research focused on investigating oxidative stress markers in individuals diagnosed with essential hypertension. It was carried out by observing patients who were already receiving routine care in a clinical setting, without any intervention or alteration in their treatment protocols.

The study population consisted of adult patients, all over 18 years of age, who were diagnosed with essential hypertension, a condition where high blood pressure is not linked to another underlying cause. The selection of participants was done using a consecutive sampling method, meaning that individuals who met the study's criteria were included as they were identified by the clinical team during the first half of 2024. This approach ensures a more straightforward and representative collection of data.

Before being enrolled, all participants were provided with clear and detailed information about the study's purpose and procedures. They gave their written informed consent, ensuring that they understood the scope of the research and agreed to participate voluntarily. In total, each participant was asked to undergo a series of medical tests designed to measure key oxidative stress markers in both blood and urine.

The primary aim of this study was to explore the relationship between these oxidative stress markers in plasma and urine, helping to understand if there is a significant correlation between these biological fluids.

### Example 1.2. Sample Collection

### • Blood Samples

To ensure consistency and minimize any confounding factors, blood samples were collected at precisely 08:00 a.m. This time was chosen because it represents a controlled setting where factors like diet or circadian rhythms have a minimal impact on the biomarkers of interest.

Participants were instructed to fast for 12 hours before the sample collection, ensuring that any dietary factors would not interfere with the results. Additionally, they were advised not to take any medication for at least 12 hours prior to the blood draw. This precaution was important because many medications can affect oxidative stress levels.

The blood was drawn via venipuncture, which is the standard procedure for collecting blood from a vein. The antecubital fossa (inner elbow) was the site of venipuncture. A tourniquet was briefly applied to help the vein become more prominent, allowing easier access for the needle. The blood was collected into a tube containing EDTA, a compound that prevents the blood from clotting, which could distort the test results.

Once collected, the blood was immediately transported to the laboratory where it underwent centrifugation. This process, performed at a speed of 1000 G at 4°C for 10 minutes, separates the plasma from the other blood components, leaving only the liquid part of the blood which contains the oxidative stress markers. The plasma was then stored at -80°C in a deep freezer to maintain its stability until it could be analyzed.

### • Urine Samples

To ensure consistency and minimize any confounding factors, urine samples were collected at precisely 08:00 a.m from the first morning spontaneous void and stored in a urine biochemistry tube with a vacuum system to prevent any contamination.

Similar to the blood samples,urine was centrifuged within 1 hour of extraction at 1000 G for 10 minutes at 4°C. The supernatant was collected and stored under the same freezing conditions as the plasma to maintain sample integrity.

### Example 1.3. Determination of TBARS in plasma (TBARS_p) or urine (TBARS_u) samples

The analysis of TBARS was a central part of this study, as these substances are produced when fats in the body undergo lipid peroxidation. Malondialdehyde (MDA) and 4-hydroxy-2-nonenal (4-HNE) are two key byproducts of this process and are recognized as markers of oxidative stress.

To measure these markers, we used a well-established method where thiobarbituric acid (TBA) is mixed with the products of lipid peroxidation. This reaction produces a colored compound that can be quantified by measuring the amount of light absorbed at a specific wavelength of 530-540 nm.

The first step was to prepare a 1% TBA solution in 20% acetic acid, adjusting its pH to 3 using sodium hydroxide. The butylated hydroxytoluene (BHT) was added to prevent the samples from oxidizing before the assay. A standard curve using MDA was created to ensure the accuracy of the measurement.

The samples were then heated at 90-95°C for 30 minutes, which is necessary to facilitate the reaction between TBA and the lipid peroxidation products. Afterward, the mixture was cooled and centrifuged to remove any particles. The resulting solution was then analyzed using a colorimetric technique that measures absorbance, allowing the concentration of TBARS in both plasma and urine samples to be determined.

Plasma concentrations were expressed in terms of nanomoles per milligram of cholesterol, while urine concentrations were normalized to creatinine levels, as creatinine excretion is relatively constant and helps account for variations in urine dilution.

### Example 1.4. Determination of Reduced Thiols

To assess the oxidation of plasma proteins, the researchers focused on measuring reduced thiols, which are sulfur-containing groups found in certain amino acids. The Ellman's reagent (DTNB) was used to detect these thiols. When it reacts with the sulfur group of thiols, a yellow-colored compound is produced, and its intensity can be measured using a spectrophotometer.

A calibration curve was created using L-cysteine, a common thiol-containing amino acid, and the absorbance of the sample was measured at 412 nm. This allowed the precise quantification of thiol groups in the plasma and urine samples.

The results were expressed as micromoles per gram of plasma protein for plasma and micromoles per milligram of urinary creatinine for urine. This normalization ensures that the findings are not skewed by variations in protein concentration or urine volume.

In summary, this observational study meticulously collected blood and urine samples from participants with essential hypertension, analyzing key biomarkers of oxidative stress. The methods employed, including standard protocols for sample collection, processing, and analysis, were carefully designed to ensure accuracy and reliability while adhering to ethical research practices.

### Example 2. Results

### Example 2.1. Statistical Analysis

The statistical analysis was performed using the SPSS 22.0 statistical program (SPSS Inc, Chicago, IL, USA), following a frequentist approach based on non-parametric statistics. The sample size was calculated using the EPIDAT software, available at https://www.sergas.es/Saude-publica/EPIDAT?idioma=es. The calculations were based on the objective of detecting a linear correlation coefficient= 0.5 between plasma and urine for TBARS and Thiols, with a 95% confidence level (95%CI) and a statistical power of at least 80% [30]. Extreme observations were corrected to 1.5 times the interquartile range (IQR) above the third quartile and below the first quartile. The absence of missing values in the variables of interest was ensured.

A preliminary descriptive approach was performed where results were expressed as number (percentage) and median (interquartile range) for qualitative and quantitative variables, respectively. The plasma-urine correlations for TBARS and Thiols were evaluated using Spearman's correlation analysis. Only results with a p-value of lower than 0.05 were deemed as relevant [9,10]. **Example 2.2. TBARS and Thiol results**

### • TBARS Results

A total of 39 patient samples were analyzed, with a median age of 64 years and a percentage of 56% women. The correlation analysis revealed a plasma-urine correlation of +0.717 (p < 0.05). The results are shown in **Table 1** and **Figure 1****.**

### • Thiol Results

A total of 40 patient samples were analyzed, with a median age of 61 years and a percentage of 55% women. The correlation analysis showed a plasma-urine correlation of +0.697 (p < 0.05). The results are shown in **Table 1** and **Figure 2****.**

A linear correlation coefficient of +0.700 suggests a strong positive correlation, meaning that as the concentration of the substance in the plasma increases, it also tends to increase in the urine, and vice versa. This indicates that the levels of the substance in these two biological fluids are closely related and that changes in one could reflect similar changes in the other.

**Table 1. Linear Correlations**

| **Correlations TBARS plasma-urine** | | | ITBARSₚ | TBARSᵤ |
|---|---|---|---|---|
| Rho Spearman | TBARSₚ | Correlation Coefficient | 1.000 | 0.717 |
| | | Sig. (bilateral) | . | < 0.001 |
| | | N | 39 | 39 |
| | TBARSᵤ | Correlation Coefficient | 0.717 | 1.000 |
| | | Sig. (bilateral) | <0.001 | . |
| | | N | 39 | 39 |

| **Correlations Thiols plasma-urine** | | | Thiolsₚ | Thiolsᵤ |
|---|---|---|---|---|
| Rho Spearman | Thiolsₚ | Correlation Coefficient | 1.000 | 0.697 |
| | | Sig. (bilateral) | . | <0.001 |
| | | N | 40 | 40 |
| | Thiolsᵤ | Correlation Coefficient | 0.697 | 1.000 |
| | | | <,001 | . |
| | | N | 40 | 40 |

## Claims

1. *In vitro* method for assessing oxidative stress comprising the identification and/or quantification of Thiobarbituric Acid Reactive Substances (TBARS) and/or thiols in a urine sample obtained from a subject, wherein the measurement of TBARS and/or thiols provides an indication of oxidative stress.

2. An *in vitro* method, according to claim 1, for assessing cardiovascular disease risk, wherein the oxidative stress assessment, based on the identification and/or quantification of TBARS and/or thiols in a urine sample, is an indication of hypertension and/or cardiovascular disease.

3. *In vitro* use of TBARS and/or thiols in a urine sample obtained from a subject for assessing oxidative stress and/or for evaluating the risk or severity of hypertension and/or other cardiovascular diseases.

4. *In vitro* method, or *in vitro* use, according to any of the preceding claims, wherein the identification and/or quantification of TBARS is performed by: (a) reacting the urine sample with thiobarbituric acid (TBA) under acidic conditions to form a colored complex, and (b) measuring the absorbance of the resulting complex, wherein the absorbance is directly proportional to the concentration of TBARS in the sample.

5. *In vitro* method, or *in vitro* use, according to any of the preceding claims, wherein the identification and/or quantification of thiols is performed by detecting reduced thiols through their reaction with 5,5'-dithiobis(2-nitrobenzoic acid) to form a colored product, wherein the absorbance is directly proportional to the concentration of reduced thiols in the sample.

6. *In vitro* method, or *in vitro* use, according to any of the preceding claims, wherein an increase in the levels of TBARS relative to a pre-established threshold value, as determined from subjects without oxidative stress, is an indication of the presence of oxidative stress in the subject.

7. *In vitro* method, or *in vitro* use, according to any of the preceding claims, wherein a decrease in the levels of reduced thiols relative to a pre-established threshold value, as determined from subjects without oxidative stress, is an indication of the presence of oxidative stress in the subject.

8. A preloaded plate system for oxidative stress assessment, comprising a plurality of wells preloaded with stabilized reagents selected from thiobarbituric acid and a thiol-detecting chromogenic compound, wherein said reagents are pre-sealed to maintain stability over an extended period, and wherein the system is configured to directly analyze urine samples without prior protein removal.

9. The preloaded plate system, according to claim 8, wherein the thiol-detecting chromogenic compound is 5,5'-dithiobis-(2-nitrobenzoic acid) (DTNB).

10. The preloaded plate system, according to any of the claims 8 or 9, wherein the stabilized reagents are sealed within the wells of the plate under vacuum to preserve their stability during storage.

11. The preloaded plate system, according to any of the claims 8 to 10, wherein the system is designed to process up to 240 urine samples in a single workday.

12. The preloaded plate system, according to any of the claims 8 to 11, wherein the system eliminates the need for blood draws and reduces the workload of healthcare personnel.

13. The preloaded plate system, according to any of the claims 8 to 12, wherein the plate is compatible with automated or high-throughput analyzers for large-scale clinical use.

14. The preloaded plate system, according to any of the claims 8 to 13, wherein the system is designed for oxidative stress marker detection in urine without additional sample preparation beyond collection.

15. The preloaded plate system, according to any of the claims 8 to 14, wherein the thiol-detecting chromogenic compound is selected from a group consisting of 5,5'-dithiobis-(2-nitrobenzoic acid) (DTNB), Ellman's reagent, and other sulfhydryl-reactive chromogenic compounds.
